# EUROPÄISCHE PATENTSCHRIFT

(11) **EP 0 505 414 B1**
(45) Veröffentlichungstag und Bekanntmachung des Hinweises auf die Patenterteilung: **02.03.1994**
(21) Anmeldenummer: 91900757.5
(22) Anmeldetag: 11.12.1990
(51) Int. Cl.: A61K 31/685

(54) **ELIMINIERUNG VON AKTIVIERTEN LYMPHOZYTEN**
KILLING OF ACTIVATED LYMPHOCYTES
ELIMINATION DE LYMPHOCYTES ACTIFS

(30) Priorität: 12.12.1989 DE 3941009
(43) Veröffentlichungstag der Anmeldung: 30.09.1992
(73) Patentinhaber: MEDMARK PHARMA GMBH, 82031 Grünwald (DE)
(72) Erfinder: WECKER, Eberhard, D-8700 Würzburg (DE); SCHIMPL, Anneliese, D-8700 Würzburg (DE); NORDSTRÖM, Rabbe, D-8022 Grünwald (DE)
(74) Vertreter: Huber, Bernhard, Dipl.-Chem.
(86) Internationale Anmeldenummer: EP9002233
(87) Internationale Veröffentlichungsnummer: WO9108746

(56) Entgegenhaltungen:
- WO-A-90/14829
- Immunobiol., vol. 156, no. 4/5, 1979, R. Andreesen et al., pp. 498-508
- Lipids, vol. 22, no. 11, 1987, Am. Oil Chemists' Soc., W.E. Berdel et al., pp. 967-999
- Blood, vol. 64, no. 6, Dec. 1984, Grune & Stratton, Inc., Plc., L. Glasser et al., pp. 1288-1291

## Beschreibung

Wesentliche Elemente des Immunsystems bei Säugetieren sind die T- und B-Lymphozyten. Während deren Entwicklung bilden diese spezifische Rezeptoren für Antigene, so daß jeder derartige Lymphozyt für den Rest seiner Lebensdauer für ein einziges Antigen Spezifität erlangt. Die ruhenden Lymphozyten (kleine Lymphozyten) einschließlich der Gedächtniszellen zirkulieren durch die Körpergewebe und Lymphorgane sowie die Körperflüssigkeiten und überwachen daher den Körper hinsichtlich des Auftretens ihrer Antigene. Tritt das spezifische Antigen auf, so erfolgt eine antigeninduzierte Lymphozyten-Proliferation unter Ausbildung der aktivierten großen Lymphozyten. Diese Proliferation läßt sich auch in vitro durch Kultivierung der Lymphoidzellen mit speziellen Antigenen darstellen. Mit dem gleichen System läßt sich auch zeigen, daß mitogene Lectine, also Proteine, welche spezifische Zelloberflächen-Determinanten, die aus Kohlehydraten bestehen, binden und vernetzen, Lymphoidzellen ebenfalls stimulieren. Eine Lymphozyten-Aktivierung durch entweder Antigene oder Mitogene führt schließlich zur Reifung des Lymphozyten zu einem Lymphoblasten. Experimentell kann die mitogene Stimulation von Lymphozyten beispielsweise durch Phytohämagglutinin (PHA) und Concavalin A (Con A) im Falle von T-Zellen, sowohl humanen als auch tierischen Ursprungs, durch Lipopolysaccharide (LPS) im Falle von B-Zellen ausgelöst werden.

Die primäre Immunantwort ist daher das Resultat einer Stimulierung der jeweils für das Antigen oder Mitogen spezifischen Lymphozyten, so daß diese aus dem ruhenden Zustand in den aktivierten Zustand übergehen, zu großen Lymphozyten werden, welche proliferieren und sich zu Effektorzellen (beispielsweise T-Zellen mit zytotoxischen oder anderen Funktionen oder Antikörper-bildenden Plasmazellen, die aus reifen B-Zellen gebildet werden) oder Gedächtniszellen differenzieren. Durch die Aktivierung der ruhenden Gedächtniszellen wird dann die sekundäre Immunantwort ausgelöst, die wiederum Effektorzellen und Gedächtniszellen hervorruft.

Durch die primäre oder sekundäre Immunantwort werden nun erwünschte wie auch unerwünschte Folgeerscheinungen bewirkt. Zu den in den meisten Fällen unerwünschten Folgeerscheinungen gehören beispielsweise Transplantatabstoßungen, allgergische Reaktionen und dgl.. Generell lassen sie sich als erworbene, durch externe Antigene verursachte immunologische Krankheiten oder Erscheinungen bezeichnen.

Wenn es gelingen würde, selektiv die Entstehung von aktivierten Lymphozyten zu eliminieren, ohne die Eigenschaften der ruhenden Lymphozyten nachteilig zu verändern, so könnte man hierdurch generell erworbene immunologische Krankheiten, die durch externe Antigene verursacht werden, unterbinden. Auf diese Weise könnten z.B. sowohl allergische Reaktionen als auch Transplantatabstoßungen unterbunden werden.

Aufgabe der Erfindung ist es daher, ein Mittel zur Verfügung zu stellen, welches spezifisch die Entstehung von aktivierten Lymphozyten zu verhindern vermag, ohne ruhende Lymphozyten zu beeinträchtigen, insbesondere ohne ruhende Lymphozyten in bezug auf ihre Stimulierbarkeit durch Antigene oder Mitogene nachteilig zu beeinflussen.

Gelöst wird diese Aufgabe durch die Verwendung von 2-Methyl-1-Octadecylglycero-(3)-phosphocholin, im folgenden als ET18OCH₃ bezeichnet, zur Herstellung eines Arzneimittels für die Eliminierung von Antigen- oder Mitogen-aktivierten Lymphozyten.

ET18OCH₃ und seine Herstellung ist bekannt. Weiter ist bekannt, daß ET18OCH₃ als Mittel gegen Tumoren (DE-A1 2619686) und gegen Multiple Sklerose (DE-A1 3530767) verwendet werden kann. Eine spezifische Eliminierung aktivierter Lymphozyten, wie sie durch die Erfindung gezeigt wird, konnte daraus nicht hergeleitet werden. Die Erfindung beruht auf der Erkenntnis, daß ET18OCH₃ selektiv durch Antigen oder Mitogen aktivierte Lymphozyten eliminiert. Selbst eine längere Einwirkung von ET18OCH₃ beeinflußt jedoch die ruhenden Lymphozyten nicht negativ und erlaubt deren nachträgliche antigene oder mitogene Stimulation. Insbesondere wurde gefunden, daß auch längere Applikation von ET18OCH₃ keinerlei negative Effekte auf die Immunreaktion in vivo ausübt und zwar sowohl für B- wie auch für T-Zell-Reaktionen.

Die erfindungsgemäße neue Indikation gestattet es daher, selektiv aktivierte große Lymphozyten zu eliminieren und damit die durch diese Lymphozyten ausgelöste Immunreaktion zu unterbinden. Da gleichzeitig die ruhenden Lymphozyten nicht negativ beeinflußt werden, wird durch das erfindungsgemäße Mittel die Wirksamkeit des Immunsystems an sich nicht beeinträchtigt. Dies stellt einen wesentlichen Vorteil gegenüber bisher bekannten immunsuppressiven Mitteln dar, die stets das gesamte Immunsystem beeinflußt haben und damit den Organismus in seiner Abwehrfähigkeit gegenüber anderen Antigenen und Mitogenen entscheidend geschwächt haben.

Die wirksame Dosis von ET18OCH₃ hängt von der Art der Verabreichung ab. Im allgemeinen werden bei der Humanapplikation Dosen zwischen 10 und 1000 mg/Person/Tag bei oraler Verabreichung angewendet. Bei dieser Applikationsweise, die beispielsweise durch Auflösen der Wirksubstanz in einem geeigneten Träger wie Milch durchführbar ist, werden vorzugweise 30 bis 300 mg/Person/Tag eingesetzt. Bei Verabreichung durch Infusion können diese Dosen noch deutlich heraufgesetzt werden. Bei topischer Applikation kommen die üblicherweise angewendeten Wirkstoffkonzentrationen auch hier in Betracht.

Die folgenden Beispiele erläutern die Erfindung in Verbindung mit der Zeichnung. In der Zeichnung stellen dar:
- Abb. I A: den Einbau von ³H-Thymidin als Maß für die Lymphozytenaktivität bei Zusatz von Zellaktivatoren
- Abb. I B: den ³H-Thymidineinbau durch Lymphozyten unter dem Einfluß von Zellaktivatoren bei Zusatz von ET18OCH₃ am ersten Tag
- Abb. I C: eine Darstellung analog I B bei ET18OCH₃-Zugabe am zweiten Tag
- Abb. II A: den Einbau von ³H-Thymidin in stimulierten Lymphozyten
- Abb. II B: eine Darstellung analog Abb. II A, jedoch bei Zusatz von ET18OCH₃ bei Zellstimulation erst nach ET18OCH₃-Verabreichung
- Abb. II C: die Kontrollergebnisse ohne Stimulation und ET180CH₃-Zusatz
- Abb. III A: zeigt den Einfluß von ET18OCH₃ auf die Entstehung von spezifischen Antikörper produzierenden Zellen
- Abb. III B: zeigt den Thymidin-Einbau durch T-Lymphozyten mit und ohne Vorbehandlung durch ET18OCH₃
- Abb. III C: zeigt analoge Ergebnisse wie in Abb. 3B bei B-Lymphozyten.

### Beispiel 1

Aus Milzzellen von Balb C Mäusen wurden die T-Lymphozyten durch Passage durch Nylonwolle isoliert. Diese wurden anschließend in Kultur mit den polyklonalen T-Zellaktivatoren Con A oder Anti-CD3-Antikörpern oder zur Kontrolle mit dem B-Zellaktivator LPS aktiviert. Jeweils der Hälfte der Kulturen wurde nach 1 oder 2 Tagen ET180CH₃ (10 µg/ml) zugesetzt. Die andere Hälfte diente als Kontrolle. Am 3. Kulturtag wurde zu allen Kulturen ³H_Thymidin zugesetzt und dessen Einbau wurde am 4. Tag gemessen.

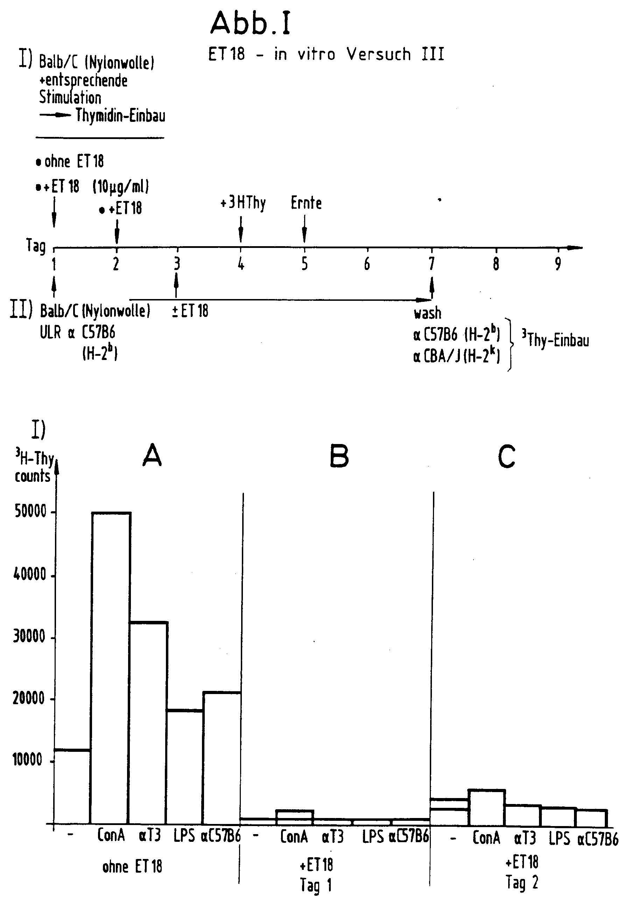

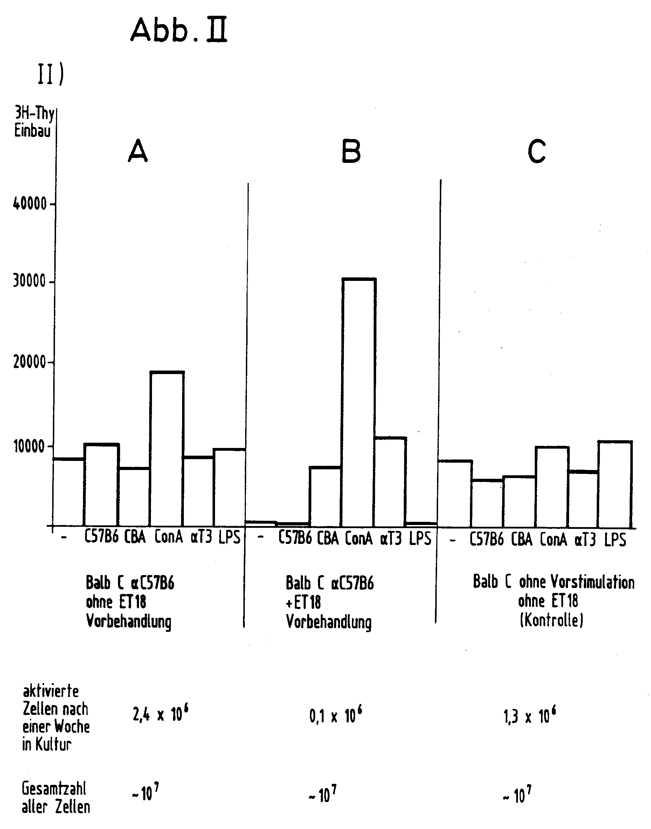

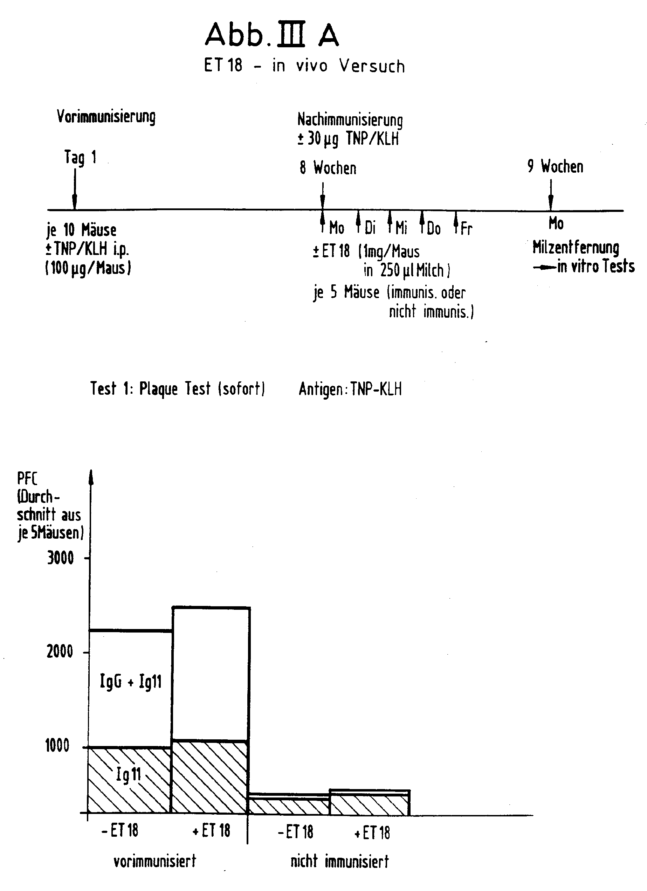

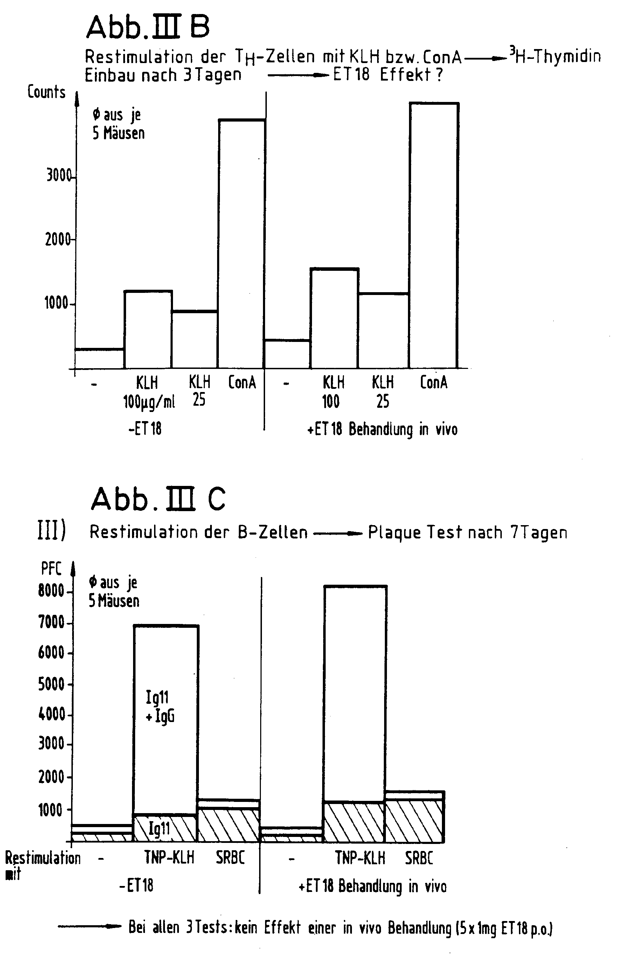

Abb. I A zeigt die zu erwartende Stimulation der Zellen in Form von erhöhtem ³H-Thymidineinbau nach polyklonaler Aktivierung mit Con A oder Anti-CD3-Antikörpern. Die geringgradige Steigerung nach Zugabe des B-Zellmitogens LPS bedeutet, daß noch restliche B-Lymphozyten in den Kulturen vorhanden waren. Alle diese Aktivierungsresultate wurden durch die Zugabe zu den Kulturen von ET180CH₃ am 1. Tag (siehe Abb. 1 B) vollkommen unterdrückt, durch Zugabe am 2. Tag (Abb. 1C) fast noch ebenso vollständig.

Daraus ergibt sich, daß ET18OCH₃ in vitro die Proliferation von T- und B-Lymphozyten der Maus praktisch vollständig verhindert. Die benutzten Aktivatoren führen nach etwa 24 Stunden zur ersten Zellteilung, so daß ET18OCH₃ zu diesem Zeitpunkt zugegeben auch das Eintreten der Zellen in erste S-Phase verhindern kann. Da jedoch auch die Zugabe von ET18OCH₃ nach 48 Stunden noch eine fast vollkommene Hemmung bewirkt, beweist dies, daß auch bereits proliferierende Zellen gegenüber ET180CH₃ hochempfindlich sind.

### Beispiel 2

Um festzustellen, ob ET18OCH₃ in geeigneten Konzentrationen dazu benützt werden kann, aus einer Population von Lymphozyten nur die aktivierten und proliferierenden Zellen spezifisch zu eliminieren, die ruhenden dagegen auszusparen, wurden folgende Versuche durchgeführt:

Durch Nylonwolle gereinigte T-Lymphozyten von Balb C Mäusen (H-2^{d}) wurden mit Milzzellen von C57B16 Mäusen, (H-2^{b}) im Sinne einer "mixed lymphocyte reaction" cokultiviert. Der Hälfte der Kulturen wurde am 3. Tag ET180CH₃ (10 µg/ml) zugesetzt, die andere Hälfte diente als Kontrolle. Eine weitere Kontrollkultur bestand aus Balb C T-Lymphozyten alleine und ohne ET18OCH₃. Nach 7 Tagen Kultur wurden alle Zellen zur Entfernung der ET18OCH₃ gewaschen und erneut in Kultur mit Zellen von C57B16 Mäusen bzw. mit Zellen von CBA Mäusen (H-2^{k}), mit den polyklonalen T-Zellaktivatoren Con A oder Anti CD3 Antikörpern oder mit dem B-Zellmitogen LPS stimuliert. Nach drei Tagen wurde dann ³H-Thymidin zugesetzt und dessen Einbau nach einem weiteren Tag bestimmt.

Abb. II A zeigt die Resultate mit den Kontrollen ohne ET18OCH₃. Dabei ist zu bemerken, daß auch die Kulturen ohne weitere Zusätze bereits einen relativ hohen background-Einbau von Thymidin zeigten, der eine weitere Zunahme nach Stimulation maskierte. Als einziges ergab Con A hier eine signifikante Erhöhung des Einbaus.

In Abb. II B sind die Ergebnisse der Kultur mit ET18OCH₃-Zusatz gezeigt. Der unspezifische background Wert ist hier völlig verschwunden, da unspezifisch proliferierende Zellen eliminiert waren. Auch die mit C57B16 Zellen restimulierten Kulturen zeigten keinen ³H-Thymidineinbau mehr. Bemerkenswert ist jedoch die Reaktion auf die neue allogene Stimulation mit CBA Zellen und vor allem die sehr gute Reaktion auf Con A und Anti CD3 Antikörper. LPS ergab keine Stimulation, da die B-Lymphozyten im Laufe der Kultur abgestorben waren.

Abb. II C zeigt die Ergebnisse mit den ganz unstimulierten Kontrollen und ohne ET180CH₃. Das Fehlen signifikanter Stimulationserfolge auch mit Con A ist dadurch zu erklären, daß unstimulierte Mäuse-Lymphozyten eine 9-tägige Kulturperiode nur schlecht überstehen.

Diese Ergebnisse zeigen, daß diejenigen T-Lymphozyten, die spezifisch durch ein Allo-Antigen, hier H-2^{b} von C57B16 Mäusen zur Proliferation aktiviert worden waren, in Anwesenheit von ET-18 vollständig eliminiert wurden. Im Gegensatz dazu blieben jedoch T-Lymphozyten anderer Antigen-Spezifitäten, die deshalb im Ruhezustand verharrten, nicht nur erhalten, sondern waren auch nachträglich noch sehr gut zur Proliferation aktivierbar. Dies wurde durch die Reaktion auf CBA, Con A und Anti CD3 Antikörper nachgewiesen. Daß ganz unstimulierte Lymphozyten (Abb. II C) auch ohne ET180CH₃ nicht mehr stimulierbar waren, zeigt, daß ET180CH₃ ruhende Zellen überhaupt nicht schädigt.

Dieser Versuch zeigt also, daß mittels ET180CH₃ Antigen-spezifisch aktivierte T-Zellklone selektiv eliminiert werden können, daß jedoch T-Zellen anderer Antigen-Spezifitäten davon nicht betroffen werden und durchaus reaktiv bleiben.

### Beispiel 3

C57b16 Mäuse wurden mit je 100 µg des klassischen Träger-Hapten-Antigens TNP-KLH (Trinitrophenyl keyhole limpet hemocyanine) immunisiert. Nach 8 Wochen erhielten die Tiere eine Booster-Injektion von je 30 µg TNP-KLH. Gleichzeitig damit und dann täglich für insgesamt 5 Tage erhielt ein Teil der Tiere 1 mg ET18OCH₃ pro Tier und Tag in Milch mittels Schlundsonde, der andere Teil Milch ohne ET18OCH₃. Eine Woche nach Boost bzw. nach der ersten ET18OCH₃-Gabe wurden die Milzen der Tiere entnommen. Die Milzen von nicht immunisierten Tieren mit und ohne ET18OCH₃ dienten als Kontrolle.

Mit einem Teil der Milzzellen wurden direkte und indirekte hämolytische Plaquetests zum Nachweis von B-Lymphozyten durchgeführt, die Anti-TNP-spezifische Antikörper der Klasse IgM oder IgG produzieren.

Abb. III A zeigt, daß die Entstehung von spezifischen Antikörper produzierenden Zellen durch ET18OCH₃ in vivo nicht beeinflußt wurde. Die Spezifität der Daten ist durch die geringe Zahl von Antikörper produzierenden Zellen bei den nicht-immunisierten Tieren, die background Werten entspricht, gezeigt.

Dieses Ergebnis zeigt, daß ET18OCH₃ keinerlei hemmende Wirkung in vivo auf die primäre oder sekundäre humorale Immunreaktion, gemessen durch die Entstehung von spezifischen Antikörper pro-duzierenden B-Lymphozyten nach Antigengaben, ausübt.

Ein weiterer Teil der oben genannen Milzzellen wurde in vitro kultiviert und mit KLH (Restimulation von T-Lymphozyten) bzw. Concanavalin A (allgemeines T-Zellmitogen) oder mit TNP-KLH (Restimulation von B-Lymphozyten) bzw. SRBC (Schaferythrozyten zur primären Stimulation von B-Lymphozyten in vitro) versetzt.

Die T-Zellreaktionen wurden im Proliferationstest durch ³H-Thymidineinbau, die B-Zellreaktionen im hämolytischen Plaquetest bestimmt.

Abb. III B zeigt den Thymidineinbau von KLH restimulierten und von Con A aktivierten T-Lymphozyten, links von Tieren ohne, rechts mit ET18OCH₃-Vorbehandlung in vivo. Dies zeigt, daß die Restimulation mit KLH dosisabhängig und spezifisch ist. Die in vivo Vorbehandlung der Tiere mit ET180CH₃ hat keinerlei negativen Effekt auf die T-Zell-Restimulation bzw. mitogene Stimulation.

Abb. III C zeigt die Ergebnisse mit B-Lymphozyten. Die Restimulation mit TNP-KLH ist spezifisch, wie auch die Primärstimulation mit SRBC. Auch hier reagieren die Zellen aus ET180CH₃ vorbehandelten Tieren mindestens so gut wie die aus Kontrolltieren. Sowohl ruhende T- als auch ruhende B-Zellen behalten daher ihre Stimulierbarkeit auch nach Verabreichung von ET18OCH₃ bei.

## Patentansprüche

1. Verwendung von 2-Methyl-1-Octadecylglycero-(3)-phosphocholin (ET18OCH3) zur Herstellung eines Arzneimittels für die vollständige Unterdrückung der Entstehung von Antigen- oder Mitogen-aktivierten T- und B-Lymphozyten.

## Claims

1. Use of 2-methyl-1-octadecylglycero-(3)-phosphocholine (ET18OCH₃) for the production of a pharmaceutical agent which completely suppresses the formation of antigen-activated or mitogenactivated T and B lymphocytes.

## Revendications

1. Utilisation de 2-méthyl-1-octadécylglycéro-(3)-phosphocholine pour la préparation d'un médicament pour la suppression totale de la production de lymphocytes T et B activés par un antigène ou un mitogène.
